(19) Europäisches Patentamt
European Patent Office
Office européen des brevets

(11) Publication number: 0 306 160
A2

(12) **EUROPEAN PATENT APPLICATION**

(21) Application number: 88307383.5

(51) Int. Cl.4: A01N 35/02 , A01N 51/00

(22) Date of filing: 10.08.88

(30) Priority: 11.08.87 GB 8719006

(43) Date of publication of application:
08.03.89 Bulletin 89/10

(84) Designated Contracting States:
AT BE CH DE ES FR GB GR IT LI LU NL SE

(71) Applicant: NATIONAL RESEARCH DEVELOPMENT CORPORATION
101 Newington Causeway
London SE1 6BU(GB)

(72) Inventor: Henderson, Ian Findlay
30 Barlings Road
Harpenden Hertfordshire AL5 2AP(GB)
Inventor: Bullock, Joseph Ian
9 Vicarage Gate Onslow Village
Guilford Surrey GU2 5RJ(GB)
Inventor: Briggs, Geoffrey Gower
46 Granby Avenue
Harpenden Hertfordshire AL5 5QR(GB)
Inventor: Larkworthy, Leslie Foot
218 Pampisford Road
South Croydon Surrey CR2 6DB(GB)

(74) Representative: Cardnell, Peter Harry Morley
Patent Department National Research Development Corporation 101 Newington Causeway
London SE1 6BU(GB)

(54) **Molluscicides.**

(57) The use as a molluscicide of a chelate of aluminium (III) with a ligand of formula I or of iron (III) with a ligand of formula II

I: $[R^1COCHCOCR^2]^-$

II: $[R^3NO.N=O]^-$

in which formulae:
$R^1$ and $R^2$, which may be identical or different, represent:
methyl, ethyl, propyl, methoxyethyl, ethoxyethyl, dimethoxy methyl or diethoxymethyl and
$R^3$ represents:
$C_1$-$C_6$ aikyl, provided that when the alkyl group contains more than four carbon atoms the group is a branched chain alkyl group.

EP 0 306 160 A2

## MOLLUSCICIDES

This invention relates to molluscicides and in particular to slug and snail poisons.

Accordingly the present invention comprises the use as a molluscicide of a chelate of aluminium (III) with a ligand of formula I or of iron (III) with a ligand of formula II

I: $[R^1COCHCOCR^2]^-$

II: $[R^3NO.N=O]^-$

in which formulae:
$R^1$ and $R^2$, which may be identical or different, represent:
methyl, ethyl, propyl, methoxyethyl, ethoxyethyl, dimethoxy methyl or diethoxymethyl and
$R^3$ represents:
$C_1$-$C_6$ alkyl, provided that when the alkyl group contains more than four carbon atoms the group is a branched chain alkyl group.

In general, chelates comprising three bidentate ligands, which may be identical or different, though which usually are identical, are preferred and especially those which lose one ligand molecule or ion to form a bis cation as shown for example in the following equation wherein acac⁻ represents $[CH_3COCH\ COCH_3]^-$.

$$Al^{III}(acac)_3 \leftrightharpoons Al^{III}(acac)_2^+ + acac^-$$
$$Al^{III}(acac)_2^+ \leftrightharpoons Al^{III}(acac)^{2+} + acac^-$$
$$Al^{III}(acac)^{2+} \leftrightharpoons Al^{3+} + acac^-$$

The ligands of formulae I and II are readily derived respectively from compounds of formula IA: $R^1COCH_2COR^2$ and IIA $R^3NOH.N=O$, by loss of a proton. In chelates comprising ligands of formula I when at least one of $R^1$ and $R^2$ is an alkyl group, the group is preferably unbranched. The $Al^{III}$ chelate in which $R^1$ and $R^2$ both represent methyl is especially preferred.

In chelates comprising ligands of formula II it is preferred that $R^3$ contains no more than four carbon atoms, $R^3$ is preferably methyl, ethyl or n-propyl and the chelate is preferably a trischelate of $Fe^{III}$ comprising identical ligands.

The following compounds of formula IA and IIA are of particular interest:

IA:
$CH_3COCH_2COCH_3$
$CH_3CH_2COCH_2COCH_3$
$CH_3CH_2COCH_2COCH_2CH_3$
$CH_3COCH_2COCH_2CH_2CH_3$
$CH_3COCH_2COCH_2OCH_3$
$CH_3COCH_2COCH(OCH_3)_2$

IIA:
$CH_3NOH.N=O$
$CH_3CH_2NOH.N=O$
$CH_3CH_2CH_2NOH.N=O$
$(CH_3)_2CHNOH.N=O$
$CH_3(CH_3)_2CH_2NOH.N=O$
$CH_3CH_2CH(CH_3)NOH.N=O$
$(CH_3)_2CH_2CH_2NOH.N=O$

The ligands and chelates hereinbefore described may be synthesised by modification of well established routes. Synthesis of complexes comprising the ligand II may be accomplished through the following route:-

$$Mg + R^3X \longrightarrow R^3MgX$$

$$R^3MgX + NO \longrightarrow \begin{array}{l} R^3\text{-}NO \ \backslash \\ \quad | \qquad MgX \\ \quad NO \ / \end{array}$$

$$\begin{array}{l} R^3\text{-}NO \ \backslash \\ \quad | \qquad MgX \\ \quad NO \ / \end{array} \xrightarrow[(2)\ Fe^{3+}]{(1)\ H^+} \left[ \begin{array}{c} R^3\text{-}NO \\ | \\ NO \end{array} \right]_3 \begin{array}{l} \backslash \\ \ Fe \\ / \end{array}$$

In the foregoing formulae X represents halogen e.g. bromine or iodine.

The present chelates of the present invention may be utilised by formulation as contact poisons or in mollusc baits.

Accordingly the present invention further includes within its scope a contact poison or a poisonous bait for molluscs comprising a molluscicidal chelate hereinbefore described.

The contact poison or poisonous bait may comprise, in addition to one or more of the present molluscicidal chelates, other components, for example other molluscicides. Such components may confer additional advantages on the contact poison or bait by, for example, synergising with the present chelates.

The present invention yet further includes with its scope a method of killing a mollusc in which the mollusc or an environment inhabited by the mollusc is treated with a molluscicidal chelate hereinbefore described.

The present chelates are of particular interest for the control of slugs because the poisons at present used, such as metaldehyde and methiocarb suffer from a disadvantage in that a proportion of the slugs in a treated population, though at first immobilised eventually recover. At least with the present $Al^{III}$ chelates comprising ligand I, recovery is generally minimal. Environmental pollution as a result of using either the aluminium or iron chelates is, furthermore, negligible.

The present chelates, and in particular the $Al^{III}$ chelates such as $Al(acac)_3$ also offer the advantage that they do not significantly affect ground beetles such as Carabidae beetles, which are attacked by other molluscicides e.g. methiocarb.

Suitable baits normally contain in addition to the molluscicidal chelate a carrier therefor and usually comprises a mollusc food such as a cereal e.g. wheatmeal, comminuted cuttle fish, starch or gelatin, which may also serve as a carrier. A mollusc phagostimulant such as a sugar e.g. sucrose, or molasses is usually included. Non nutrient carriers of interest include non nutrient polymeric materials, pumice, carbon and materials useful as carriers for insecticides. The bait usually comprises a binder, which is suitably waterproof, such as paraffin wax or casein and may advantageously comprise a bird repellent, for example a blue colourant. In order to inhibit deterioration of the bait a fungistat may be included.

Typically the bait contains at least 3% by weight and no more than 16% by weight of molluscicide. At least when the molluscicide is an aluminium chelate the preferred concentration in the bait is 10-13% by weight.

When used as a contact poison the molluscide is typically formulated as a dust or spray, for application to foliage, soil, stubble or trash (plant residues). Examples of solid carriers include talc, chalk bentonite, clay and the like and examples of liquid carriers include water (if necessary with an emulsifier), alcohols e.g. lower alcohols such as methanol or ethanol, ketones e.g. lower ketones such as acetone or methyl ethyl ketone, liquid hydrocarbons and the like.

The treatment of both terrestrial and aquatic molluscs in accordance with the present invention is envisaged, the species Deroceras reticulatum, Arion hortensis, Milax budapestensis, Cepaea hortensis, Helix aspersa and Achatina spp being of particular interest as targets.

The invention is illustrated by the following Examples:

## EXAMPLES

Laboratory Tests

Test animals

All laboratory tests were made with field-collected grey field slugs, Deroceras reticulatum (Pulmonata, Limacidae) and also with Arion hortensis in the weight range 0.4-0.6g. They were held in polythene bowls on wet filter paper in a controlled environment cabinet cycling 12 hours light at 15 °C and 12 hours dark at 5 °C. Voluntary feeding tests were made under these conditions but all other laboratory bioassays were carried out at 10 °C constant temperature with an overnight pre-treatment period also at 10 °C.

Example 1

Contact with coated glass plate

Groups of ten slugs were placed foot-down on sheets of glass, 10 cm x 10 cm, previously coated with an aqueous solution of suspension of test chemical and air dried to leave a deposit of known weight per unit area. Slugs were confined to a treated area for a period of 50 minutes at 10 °C on plates with increasing surface loadings, then removed and held in wet filter paper-floored petri dishes with food. The number of dead slugs after seven days was recorded and the values used to calculate median lethal surface concentrations ('$LC_{50}$') for each compound by probit analysis.

Results are shown in Table 1

Example 2

Contact with treated soil surface

Groups of ten slugs were held in plastic seed trays, 20 x 34 cm x 5 cm deep, filled to a depth of 1 cm with standard soil, a sandy clay loam from Rothamsted Farm, with a pH in water of 5.2, previously oven-dried, sieved (2 mm mesh), and rewetted to field capacity in the trays. Test chemicals were mixed with 5g dry soil, ground together and sprinkled on to the wet soil surface. Slugs were confined to the treated surface by a 16 v pulsed DC electric fence attached to the tray sides. Trays were covered to maintain a high Relative Humidity and food was provided. Dead slugs were removed daily and the final mortality count made after 10 days.

Results as shown in Table 1.

Examples 3-13

Stomach poison action, voluntary feeding

Test chemicals were offered to slugs at a range of concentrations in a wheat-meal based formulation, groups of ten slugs being confined in 15 cm crystallising dishes containing only wet filter paper and a solid watch glass filled with 3 g (dry weight) of test bait for four 24-hour cycles of the 5 °C dark/15 °C light regime and noting weight eaten and number of animals dead after 7 days.

Results are shown in Tables II and III.

TABLE 1

Comparative toxicities of aluminium compounds on glass and on wet soil surfaces. Calculated $LC_{50}$ values of individual tests and their mean.

| | | $LC_{50}$($\mu$g metal $cm^2$) | |
|---|---|---|---|
| Compound | Test No. | On glass (50 min). | On soil (10 days) |
| Aluminium sulphate | 1 | (<40) | 82.0 |
| | 2 | 15.2 | 191.2 |
| | 3 | 17.7 | 383.9 |
| | 4 | 29.7 | 172.5 |
| | Mean | 20.9 | 207.4 |
| $Al^{III}$ acetylacetonate | 1 | (10-100)(Example 1) | (10-100)(Example 2) |
| | 2 | (10-100) | 45.3 |
| | 3 | | 22.9 |
| | 4 | - | 34.0 |
| | Mean | (< 100) | 34.1 |

TABLE II

Effect of form and concentration of aluminium on consumption of wheatmeal bait and on slug mortality.

| Bait eaten by 10 slugs (g Dry Wt) | | | Mortality after 7 days ($^x$/10) | |
|---|---|---|---|---|
| % Al | As aluminium sulphate | As aluminium acetylacetonate | (As aluminium sulphate) | (As aluminium acetylacetonate) |
| 0 | 0.53 | - | 3 | - |
| 0.01 | 1.31 | 1.46 (Ex.3) | 1 | 1 |
| 0.03 | 1.08 | 0.95 (Ex.4) | 2 | 1 |
| 0.1 | 0.62 | 0.80 (Ex.5) | 3 | 2 |
| 0.3 | 0.89 | 0.51 (Ex 6) | 3 | 4 |
| 1.0 | 0.41 | 0.43 (Ex.7) | 5 | 10 |
| 3.0 | 0.42 | 0.52 (Ex.8) | 2 | 10 |

TABLE III

Effect of metal ion concentration on consumption of aluminium acetylacetonate wheatmeal bait and on slug mortality.

| % Metal | Bait eaten by 10 slugs (g Dry Wt.) | Mortality after 9 days ($^x$/10) |
|---|---|---|
| 0 | 1.800 | 0 |
| 0.1 | 0.315 (Ex. 9) | 4 |
| 0.5 | 0.105 (Ex. 10) | 8 |
| 1.0 | 0.120 (Ex. 11) | 8 |
| 5.0 | 0.180 (Ex. 12) | 8 |
| 10.0 | 0.090 (Ex. 13) | 8 |

Example 14

Field Trials

Field experiments were made on a prepared site on Rothamsted Farm, previously down to grass/clover and irrigated to increase slug numbers, and subsequently direct-drilled with winter wheat (5.11.85) after removing most of the herbage by forage harvester (16.8.85) and spraying with glycophosphate to kill the regrowth (11.9.85). The resulting soil surface was relatively undisturbed and had very little trash cover, facilitating observation of surface applications and slugs remaining above ground.

Example 14

Field experiment 1

Treatments to control slug damage to direct-drilled winter wheat, cv. 'Avalon', were tested on plots 6 m x 8 m in four randomised blocks separated by 10 m internal headlands. Methiocarb 4 per cent bait (Draza pellets) at 5.5 kg $ha^{-1}$, and aluminium acetylacetonate 12 per cent experimental bait as 11 kg $ha^{-1}$, both produced dead and immobile slugs visible on the soil surface for several days following application on 29.12.85. They were counted (four 1 m x 1 m quadrats per plot) on the day following application and again on the third day after.

Results are shown on Table IV.

TABLE IV

Field experiment 1. Numbers of slugs observed dead or immobile on plot surface one and three days after treatment with methiocarb (Draza) pellets and with an experimental molluscicide (4 quadrats 1 $m^2$ per plot x 4 plots).

| | Slugs Observed Dead or Immobile | | | |
|---|---|---|---|---|
| | + 1 Day | | + 3 Days | |
| Treatment | Total | n $m^{-2}$ ±SE | Total | n $m^{-2}$ ±SE |
| Methiocarb bait (Draza) @ 5.5 kg $ha^{-1}$ | 142 | 8.9 ± 0.94 | 189 | 11.8 ± 2.28 |
| Aluminium acetyl-acetonate bait @ 11.0 kg $ha^{-1}$ | 374 | 23.4 ± 5.05 | 214 | 13.4 ± 2.64 (Ex. 14) |

Examples 15 and 16

Field experiment 3

The number of slugs killed or immobilised by proprietary 4 per cent methiocarb bait (Draza pellets), methiocarb 4 per cent experimental bait, aluminium acetylacetonate 4 per cent experimental bait and 12 per cent experimental bait, all applied at 5.5 kg $ha^{-1}$ to 0.5 m x 1 m areas of the winter wheat field, was compared over a 14-day period from 4.1.86. Plots were separated by 5 m. Each treatment was replicated eight times in two 4 x 4 latin squares. Dead or immobile slugs were again collected daily and held for 7 days at 10 °C.

Results are shown in Table V.

Notes on the results

A. Laboratory tests.

Preliminary tests exposing slugs to simple metal salts on a glass surface indicated significant toxicity in several metals, including Copper, Zinc, Nickel, Iron and Aluminium. More detailed tests on glass plates were made with the last as its use in the field would not leave undesirable residues in the soil. The results of holding slugs in contact with increasing surface loadings of aluminium in two different chemical compounds on a glass surface for 50 minutes and on a soil surface for 10 days are given in Table 1. Mortality is expressed as a mediam lethal concentration or '$LC_{50}$' as $\mu g$ metal ion $cm^{-2}$. The results are given as $LC_{50}$ values with their 95 per cent confidence intervals for individual tests together with the mean $LC_{50}$ for all tests. Where no $LC_{50}$ value could be calculated because of incorrect choice of does range the estimated value is given in brackets.

As the sulphate on glass aluminium gave $LC_{50}$ values around 20 $\mu g$ metal $cm^{-2}$.

## TABLE V

Field experiment 3. Numbers of dead or immobilised slugs collected from plots treated with methiocarb or aluminium acetylacetonate baits at 5.5 kg $ha^{-1}$, and numbers which subsequently died after holding at 10°C for a further 7 days.

| Bait | | Daily post treatment catch 1 | 2 | 3 | 4 | 5 | 6 | 7 | 8 | 9 | 10 | 11 | 12 | 13 | 14 | Total |
|---|---|---|---|---|---|---|---|---|---|---|---|---|---|---|---|---|
| Methiocarb 4x ('Draza pellets) | Collected | 0 | 2 | 0 | 2 | 38 | 3 | 11 | 2 | 1 | 0 | 0 | 0 | 0 | 0 | 59 |
| | Died | 0 | 2 | 0 | 2 | 23 | 1 | 4 | 2 | 1 | | | | | | 35 |
| Methiocarb 4x (experimental bait) | Collected | 0 | 1 | 0 | 5 | 28 | 4 | 6 | 2 | 3 | 0 | 0 | 0 | 0 | 0 | 47 |
| | Died | 0 | 1 | 0 | 4 | 12 | 2 | 3 | 1 | 1 | | | | | | 24 |
| Example 15 Aluminium acetylacetonate 4x (experimental bait) | Collected | 0 | 2 | 0 | 7 | 12 | 0 | 0 | 0 | 0 | 0 | 0 | 0 | 0 | 0 | 21 |
| | Died | 0 | 2 | 0 | 7 | 6 | 0 | 0 | 0 | 0 | | | | | | 15 |
| Example 16 Aluminium acetylacetonate 12x (experimental bait) | Collected | 2 | 8 | 0 | 9 | 54 | 0 | 13 | 2 | 3 | 0 | 0 | 0 | 0 | 0 | 91 |
| | Died | 2 | 7 | 0 | 9 | 54 | 0 | 11 | 1 | 3 | | | | | | 87 |

When applied to a wet soil surface the activity of all the compounds was greatly reduced despite the increase in exposure time from 50 minutes to 10 days. The reduction in activity was not the same for all compounds.

When offered sweetened wheatmeal bait containing increasing concentrations of aluminium as sulphate or acetylacetonate in non-choice tests the amount eaten fell with increasing concentration. Similar weights of bait were eaten regardless of the form of aluminium used, but mortality was much greater with the chelated aluminium bait, reaching 100 per cent at 1 per cent Aluminium content (Table II).

When offered sweetened wheatmeal bait containing aluminium acetylacetonate over a range of concentrations of metal ion up to 10 per cent, consumption was again depressed with increasing proportion of poison. The optimum concentration of aluminium acetylacetonate lay in the range 0.5 to 5.0 per cent of aluminium (Table III).

B. Field Experiment 1

The mean number of slugs dead or immobilised on the surface of the 6 x 8 m plots on the day following treatments begun on 29 October suggests than the aluminium acetylacetonate experimental bait at 11 kg $ha^{-1}$ was the most effective treatment, catching 23.4 slugs $m^{-2}$. Commercial methiocarb 4 per cent bait (Draza pellets) at the manufacturers' recommended rate of 5.5 kg $ha^{-1}$ caught 8.9 slugs $m^{-2}$. A second count after a further two days had passed confirmed this order although the differences were less. The results of the second count are less reliable. (Table IV).

Field Experiment 3

In this comparison of methiocarb and aluminium acetylacetonate, all bait was applied at the same rate, 5.5 kg $ha^{-1}$, and the two poisons were also compared in the same experimental bait carrier at the same concentration of poison. Weather adverse to slug activity and the lower application rate reduced the numbers of slugs caught overall. 4 per cent methiocarb as Draza caught more slugs then 4 per cent methiocarb in the experimental bait, but 12 per cent aluminium acetylacetonate (1 per cent Aluminium) in experimental bait, caught most slugs. On holding for recovery only 4/91 (4.4 per cent) of the slugs which ingested the 12 per cent aluminium acetylacetonate bait resumed normal activity, although at the lower concentration (3 per cent ai) 6/21 (28.6 per cent) did so.

Of the methiocarb (experimental bait) - poisoned slugs 23/47 (48.9 per cent) recovered, while 24/59 (40.7 per cent) of those poisoned by methiocarb (Draza bait) did so (Table V).

Example 17

Laboratory tests where 3 replicates of 10 slugs were confined with standard baits containing 1% metal ion in different compounds, and no alternative food supplied, gave the following results:
Aluminium sulphate ($Al_2(SO_4)_3$): 0/10, 0/10, 0/10 killed
Aluminium acetylacetonate: 10/10, 10/10, 10/10 killed
(Ex 17)

Example 18

A field trial, where baits were placed among a growing wheat crop and the numbers of slugs 'caught' recorded over 11 days, gave these results:

| | |
|---|---|
| Methiocarb 4% (Bayer 'Draza' pellets) | 21 caught |
| " " (RES Standard Bait)* | 19 " |
| Metaldehyde 6% (Pan Britannica Ind. Mini pellets) | 32 " |
| " " (RES Std. Bait)* | 50 " |
| $Al_2(SO_4)_3$ (1% Aluminium) (RES Std. Bait)* | 0 " |
| $Al(acac)_3$ (" " ) ( " " " ) | 41 " (Ex 18) |

*RES: Rothamsted Experiment Station: See Examples 19 - 22

Examples 19-22

Bioassay Method

Test compounds were incorporated into a standard wheat-flour-based edible carrier containing 10% paraffin wax binder and 2.5% sucrose phagostimulant, in increasing concentrations 0, 1, 4, 7, 10, 13 and 16% a.i.

Groups of ten field-collected D. reticulatum in the weight range 400-600 mg were confined with 0.5 g DM test bait at each concentration for a period of 4 days under a regime of 12 h dark @ 5° C and 12 light @ 15° C. Weight of bait eaten and number of slugs killed were recorded. Each group of compounds was tested against a concurrently run $Al(ac.ac)_3$ standard bait.

The results are shown in Table VI. In Examples 23 to 35, where six groups of ten slugs are offered bait containing 1,4,7,10,13 or 16% a.i., have been reduced to two values, (i), the total amount of a.i. ingested (mg) which gives an indication of the palatability of the compound, and (ii), the total number of slugs killed (x/60) which, with (i), gives an indication of its relative toxicity.

In Table VII there is presented a comparison of the efficacy of present iron and aluminium chelates with metaldehyde and methiocarb under field conditions (grass/clover).

## TABLE VI

Effect of increasing concentrations of iron and aluminium chelates in a standard bait on feeding and kill of Deroceras reticulatum

| Example | Compound | a.i. (%) | Eaten (%) | Kill (x/10) |
|---|---|---|---|---|
| 19 | Tris (2,4-pentanedionato) Al III | 0 | 100 | 0 |
| | | 1 | 100 | 0 |
| | | 4 | 44 | 0 |
| | | 7 | 29 | 6 |
| | 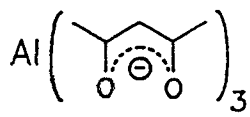 | 10 | 20 | 7 |
| | | 13 | 19 | 8 |
| | | 16 | 20 | 8 |
| 20 | Tris (2,4-hexanedionato) Al III | | 100 | 0 |
| | | | 100 | 1 |
| | | | 100 | 0 |
| | 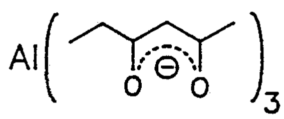 | | 58 | 4 |
| | | | 29 | 1 |
| | | | 26 | 5 |
| | | | 22 | 5 |
| 21 | Tris (3,5-heptanedionato) Al III | | 100 | 0 |
| | | | 100 | 1 |
| | | | 100 | 3 |
| | | | >10 | 0 |
| | 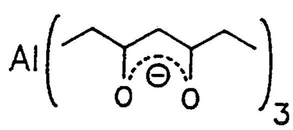 | | >10 | 1 |
| | | | >10 | 1 |
| | | | >10 | 2 |
| 22 | Tris (N-nitroso-N-methyl hydroxylaminato) Fe III | | 100 | 2 |
| | | | 17 | 5 |
| | | | 11 | 8 |
| | | | 4 | 5 |
| | 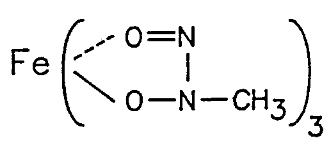 | | 8 | 5 |
| | | | 5 | 7 |
| | | | 7 | 8 |

| Example | Compound | a.i.ingested (mg/60 slugs) | Slugs dead (x/10) |
|---|---|---|---|
| 23 | Tris(2,4-pentanedionato) Al III 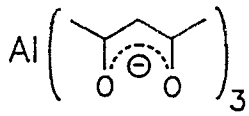 | 61 ±4.6 | 35 ±1.4 |
| 24 | Tris (2,4-hexanedionato) Al III 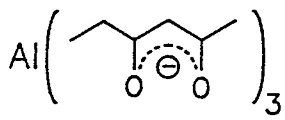 | 94 | 16 |
| 25 | Tris(3,5-heptanedionato) Al III 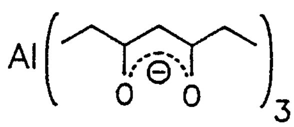 | 15 | 8 |
| 26 | Tris (2,4-heptanedionato) Al III 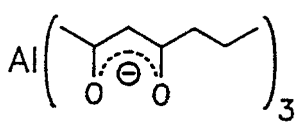 | 21 | 4 |
| 27 | Tris (1-methoxy-2,4-pentane dionato) Al III 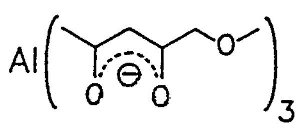 | 40 | 10 |
| 28 | Tris (N-nitroso-N-methyl hydroxylaminato) Fe III 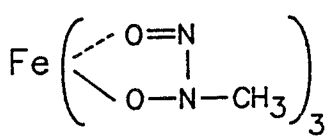 | 17 | 38 |

| Example | Compound | a.i.ingested (mg/60 slugs) | Slugs dead (x/10) |
|---|---|---|---|
| 29 | Tris (1-dimethoxy-2, 4-pentanedionato) Al III 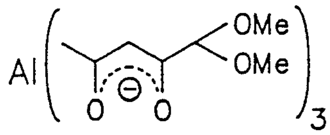 | 35 | 14 |
| 30 | Tris (N-nitroso-N-ethyl hydroxylaminato) Fe III 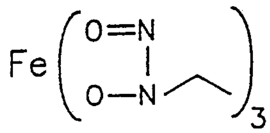 | 6 | 49 |
| 31 | Tris (N-nitroso-N-n-propyl hydroxylaminato) Fe III 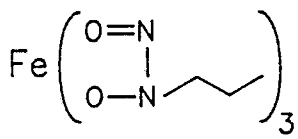 | 50<br>55 | 58<br>58 |
| 32 | Tris (N-nitroso-N-2-propyl hydroxylaminato) Fe III 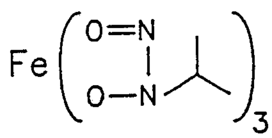 | 3 | 43 |
| 33 | Tris (N-nitroso-N-n-butyl hydroxylaminato) Fe III 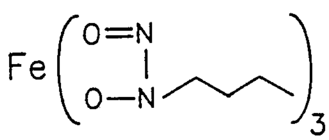 | 53 | 37 |
| 34 | Tris (N-nitroso-N-2-butyl hydroxylaminato) Fe III 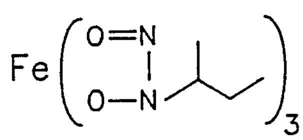 | 54 | 46 |

| Example | Compound | a.i.ingested (mg/60 slugs) | Slugs dead ($^{x}/_{10}$) |
|---|---|---|---|
| 35 | Tris (N-nitroso-N-2-methylpropyl hydroxylaminato) Fe III 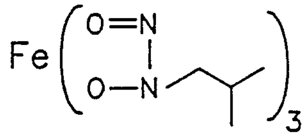 | 160 | 42 |

TABLE VII

| Example No. | Bait Poison | % ai | % sucrose | Total Slugs 'Dead' or 'Immobilised' (Cumulative) | | | | | | | | |
|---|---|---|---|---|---|---|---|---|---|---|---|---|
| | Compound of Example No. | | | Day 1 | | Day 2 | | Day 3 | | Day 4 | | Total |
| | | | | D | I | D | I | D | I | D | I | D+I |
| 36 | 28 | 10 | 2.5 | 81 | 4 | 117 | 19 | 137 | 34 | 162 | 40 | 202 |
| 37 | 30 | 10 | 2.5 | 127 | 77 | 194 | 33 | 254 | 51 | 313 | 54 | 367 |
| 38 | 31 | 10 | 2.5 | 103 | 30 | 137 | 52 | 165 | 75 | 193 | 82 | 275 |
| 39 | 33 | 10 | 2.5 | 22 | 43 | 41 | 60 | 47 | 89 | 58 | 89 | 147 |
| 40 | 34 | 10 | 2.5 | 37 | 34 | 42 | 48 | 42 | 85 | 45 | 86 | 131 |
| - | Metaldehyde | 10 | 2.5 | 126 | 33 | 139 | 142 | 241 | 161 | 271 | 190 | 461 |
| - | Metaldehyde (PP pellets) | 6 | - | 38 | 137 | 46 | 224 | 69 | 317 | 86 | 368 | 454 |
| - | Methiocarb | 10 | 2.5 | 9 | 244 | 10 | 371 | 11 | 499 | 16 | 541 | 557 |
| - | Methiocarb (Draza pellets) | 4 | - | 5 | 195 | 9 | 197 | 12 | 331 | 14 | 390 | 404 |
| 41 | Al $(acac)_3$ | 10 | 2.5 | 210 | 13 | 294 | 54 | 378 | 86 | 435 | 98 | 533 |
| 42 | Al $(acac)_3$ | 10 | 5 | 185 | 11 | 266 | 27 | 354 | 57 | 419 | 66 | 485 |
| 43 | Al $(acac)_3$ | 10 | 10 | 186 | 7 | 276 | 26 | 338 | 51 | 396 | 64 | 460 |
| - Results of first 4 days of trial | | | | | | | | | | | | |

## Claims

1. The use as a molluscicide of a chelate of aluminium (III) with a ligand of formula I or of iron (III) with a ligand of formula II

I: $[R^1COCHCOCR^2]^-$

II: $[R^3NO.N=O]^-$

in which formulae:
$R^1$ and $R^2$, which may be identical or different, represent:
methyl, ethyl, propyl, methoxyethyl, ethoxyethyl, dimethoxy methyl or diethoxymethyl and
$R^3$ represents:
$C_1$-$C_6$ alkyl, provided that when the alkyl group contains more than four carbon atoms the group is a branched chain alkyl group.

2. A use according to Claim 1, in which the chelate comprises three identical bedentate ligands.

3. A use according to Claim 1, in which the chelate is of aluminium (III) with a ligand of formula I as hereinbefore defined.

4. A use according to Claim 3, in which at least one of $R^1$ and $R^2$ is an unbranched alkyl group.

5. A use according to Claim 3, in which the chelate comprises a ligand formed by loss of a proton from one of the following compounds:

$CH_3COCH_2COCH_3$
$CH_3CH_2COCH_2COCH_3$
$CH_3CH_2COCH_2COCH_2CH_3$
$CH_3COCH_2COCH_2CH_2CH_3$
$CH_3COCH_2COCH_2OCH_3$
$CH_3COCH_2COCH(OCH_3)_2$

6. A use according to Claim 1 in which the chelate is of formula $Al^{III}[CH_3COCHCOCH_3]_3$.

7. A use according to Claim 1, in which the chelate is of iron (III) with a ligand of formula II as hereinbefore defined.

8. A use according to Claim 7, in which $R^3$ contains no more than four carbon atoms.

9. A use according to Claim 7, in which $R^3$ is methyl, ethyl, or n-propyl.

10. A use according to Claim 7, in which the chelate comprises a ligand formed by loss of a proton from one of the following compounds:

$CH_3NOH.N=O$
$CH_3CH_2NOH.N=O$
$CH_3CH_2CH_2NOH.N=O$
$(CH_3)_2CHNOH.N=O$
$CH_3(CH_3)_2CH_2NOH.N=O$
$CH_3CH_2CH(CH_3)NOH.N=O$
$(CH_3)_2CH_2CH_2NOH.N=O$

11. A use according to Claim 7, in which the chelate is of formula: $Fe^{III}[CH_3NON=O]_3$ or $Fe^{III}$-$[CH_3CH_2NON=O]_3$ or $Fe^{III}[CH_3CH_2CH_2NON=O]_3$.

12. A contact poison or poisonous bait for molluscs, comprising a molluscicidal chelate as hereinbefore defined.

13. A poison bait for molluscs according to Claim 12, which comprises a chelate molluscicide as hereinbefore defined and a carrier therefor.

14. A poison bait according to Claim 12 or 13, which comprises a mollusc food.

15. A poison bait according to Claim 14, in which the mollusc food is a cereal, comminuted cuttle fish, mollases or gelatin.

16. A poison bait according to any of Claims 12 to 15 which comprises a mollusc phagostimulant.

17. A poison bait according to Claim 16, in which the phagostimulant is a sugar or starch.

18. A poison bait according to Claims 12 or 13 which comprises a non nutrient carrier.

19. A poison bait according to Claim 18, in which the non nutrient carrier is an artificial polymer, pumice, carbon or a material useful as a carrier for an insecticide.

20. A poison bait according to any of Claims 12 to 19, which comprises a binder.

21. A poison bait according to Claim 20, in which the binder is paraffin wax or casein.

22. A poison bait according to any of Claims 12 to 21, which comprises a bird repellent.

23. A poison bait according to Claim 22, in which the bird repellent is a blue colourant.

24. A poison bait according to any of Claims 12 to 23, which comprises a fungistat.

25. A poison bait according to any of Claims 12 to 24, which comprises at least 3% by weight of a molluscicidal chelate as hereinbefore defined.

26. A poison bait according to any of Claims 12 to 25, which comprises no more than 16% by weight of a molluscicidal chelate as hereinbefore defined.

27. A poison bait according to any of Claims 12 to 26, which comprises 10 - 12% by weight of a molluscicidal chelate as hereinbefore defined.

28. A poison bait according to any of Claims 12 to 27 in which the chelate is of formula $Al^{III}$ - $[CH_3COCHCOCH_3]_3$.

29. A process for the production of a poison bait for molluscs in which a chelate molluscicide as hereinbefore defined is formulated with a carrier therefor.

30. A method of killing a mollusc in which the mollusc or an environment inhabited by the mollusc is treated with a molluscicidal chelate hereinbefore defined.

31. A method according to Claim 30, in which an environment inhabited by the mollusc is treated with a poison bait comprising a chelate hereinbefore defined.